# EUROPÄISCHE PATENTANMELDUNG

(11) **EP 3 120 810 A1**
(43) Veröffentlichungstag der Anmeldung: **25.01.2017**
(21) Anmeldenummer: 16175929.5
(22) Anmeldetag: 23.06.2016
(51) Int. Cl.: A61F 2/24, A61B 8/00, A61B 34/20, A61B 5/00, A61B 5/06

(54) **KATHETERSYSTEM ZUR LOKALISIERUNG UND IMPLANTATION EINES KÖRPERTEILERSATZES**

(30) Priorität: 21.07.2015 DE 102015111783
(71) Anmelder: Biotronik AG, 8180 Bülach (CH)
(72) Erfinder: Bartels, Marc, 10115 Berlin (DE); Ulmer, Jens, 8700 Küsnacht (CH); Kämpf, Udo, 13595 Berlin (DE)
(74) Vertreter: Galander, Marcus

(57) **Zusammenfassung**

Die Erfindung beschreibt ein Kathetersystem umfassend mindestens einen Katheterschaft 3, ein anisotropes Implantat, insbesondere eine implantierbare Herzklappenprothese 6, ein Steuergerät zur Steuerung und Manipulation des Katheters sowie mindestens drei Elektroden 4, 5 und 11 im distalen Bereich des Katheters, die über jeweils eine Elektrodenleitung leitend mit einer Analyseeinheit verbunden sind. Das Kathetersystem kann zwischen einem Einführzustand und einem Orientierungszustand wechseln, wobei dieser Wechsel durch eine Manipulation des Steuergeräts ausgelöst wird. Im Orientierungszustand spannen die drei Elektroden des Kathetersystems einen anderen Raumwinkel auf als im Einführzustand.

## Beschreibung

Die vorliegende Erfindung betrifft ein Kathetersystem umfassend mindestens einen Katheterschaft, ein anisotropes Implantat, ein Steuergerät zur Steuerung und Manipulation des Katheters sowie mindestens drei Elektroden im distalen Bereich des Katheters, die über jeweils eine Elektrodenleitung leitend mit einer Analyseeinheit verbunden sind. Die Erfindung wird im Folgenden anhand einer implantierbaren Herzklappenprothese zum Ersatz der natürlichen Mitralklappe beschrieben, ist jedoch nicht darauf eingeschränkt. Die Erfindung betrifft jedes Kathetersystem mit einem anisotropen Implantat wie eingangs beschrieben. Dies können Implantate wie Occluder Devices, Stents für Bifurkationen oder an sich symmetrische Implantate mit einer künstlichen Anisotropie (wie beispielsweise ein Marker) sein. Entscheidend ist, dass das Implantat eine Anisotropie aufweist. Die Anisotropie besteht dabei bezüglich einer Rotation um die Hauptachse des Implantats. Als Hauptachse des Implantats wird im Rahmen dieser Erfindung die Längsachse des Implantats verstanden, die im Einführzustand des Implantats auf der Katheterachse liegt.

Herzklappen können durch verschiedene Ursachen in ihrer Funktionalität eingeschränkt sein. Im Falle der Aorten- oder Mitralklappe kommen sowohl erworbene Aorten- oder Mitralklappenstenose als auch eine Aorten- oder Mitralklappeninsuffizienz in Frage. Eine Klappenstenose kann durch eine Entzündung entstehen, ist jedoch häufiger das Resultat einer fortschreitenden Sklerose (umgangssprachlich auch Verkalkung) der Klappe, die zu einer Degeneration und Kalzifizierung der Klappen und allmählich zu einer Stenose führen. Die Stenose führt vereinfacht ausgedrückt zu einer Verengung der Öffnungsfläche der Klappen, wodurch zunehmend ein höherer Druck notwendig wird, um die gleiche Menge Blut durch die Klappen zu befördern. Bei weiterem Fortschreiten der Erkrankung kommt das Herz letztlich an seine Grenzen und kann nicht mehr hinreichend Blut fördern, der Patient ist in seinen Aktivitäten eingeschränkt.

Ähnliche Symptome treten bei einer Klappeninsuffizienz auf. In derartigen Fällen schließen die Klappen nicht mehr vollständig und es kommt zu einer Rückströmung des geförderten Blutes. Der physiologische Effekt für den Patienten ist ähnlich, es kann nicht mehr genug Blut über die geschädigte Klappe gefördert werden.

Wenn durch die Klappenschädigung die Pumpleistung des Herzens zu weit absinkt, müssen die geschädigten Klappen ersetzt werden. Hierzu gibt es im Stand der Technik im Wesentlichen zwei verschiedene Verfahren.

Der Einsatz einer künstlichen Herzklappenprothese als Ersatz der Aorten- oder Mitralklappe kann in einem offenen, chirurgischen Verfahren erfolgen. Eine derartige Operation erfolgt in Vollnarkose. Der Burstkorb des Patienten wird eröffnet, das Herz wird vom Blutkreislauf abgetrennt und die Blutversorgung des Patienten erfolgt über eine Herz-Lungen Maschine. Anschließend wird die erkrankte Aorten- oder Mitralklappe chirurgisch entfernt und durch das Implantat ersetzt und schließlich das Herz wieder in den Blutkreislauf integriert, der Patient von der Herz-Lunge Maschine befreit.

In der letzten Zeit hat sich ein katheterbasiertes, minimal invasives Verfahren als Alternative zur offenen chirurgischen Technik etabliert. Dieses Verfahren kommt vor allem bei älteren und geschwächten Patienten zur Anwendung, die nicht mehr dem Risiko einer Vollnarkose ausgesetzt werden sollen. Die dafür verwendeten Herzklappenprothesen bestehen im Wesentlichen aus einem Grundgerüst und einer darin befestigten Klappenanordnung.

Die Implantation der Herzklappenprothese erfolgt über einen Katheter, der beispielsweise über die Leistenarterie eingeführt wird. Die Herzklappenprothese wird dann über diesen Katheter an den Implantationsort gebracht. Dort wird die Herzklappenprothese entfaltet und im Gefäß an der Position der natürlichen Herzklappe verankert. Die natürliche Herzklappe wird dabei nicht entfernt sondern lediglich von der Herzklappenprothese verdrängt.

Zum Einbringen mittels Katheter muss die Herzklappenprothese entsprechend auf dem Schaft des Katheters angebracht werden. Dabei muss zum Einbringen der Durchmesser der Herzklappenprothese deutlich kleiner sein als am Implantationsort. Entsprechend wird die Herzklappenprothese auf den Katheterschaft komprimiert ("gecrimpt") und nach dem Einbringen am Implantationsort expandiert. Die Expansion kann dabei wie bei einem Stent selbstexpandierend oder durch Expansion eines Ballons erfolgen, je nach Grundgerüst.

Einen derartigen Katheter und eine derartige Herzklappenprothese zum Ersatz der natürlichen Aortenklappe wird beispielsweise in EP 2 260 796 beschrieben.

Die Mitralklappe oder auch Bikuspidalklappe (Valva atrioventricularis sinistra, Valva mitralis oder Valva bicuspidalis) befindet sich zwischen linkem Vorhof und linker Herzkammer (linker Ventrikel), wo sie den Rückfluss von Blut aus der linken Herzkammer in den linken Vorhof bei der Kontraktion der Kammer verhindert. Ihre Form ähnelt einer Mitra (Bischofsmütze). Die Bezeichnung Bikuspidalklappe leitet sich von den zwei Segeln (lateinisch cuspides) ab, aus denen diese Segelklappe besteht. Die natürliche Mitralklappe weist keinen rotationssymmetrischen Querschnitt auf, sondern einen anisotropen Querschnitt vergleichbar mit einem D.

Bei dem Ersatz einer Mitralklappe steht man daher grundsätzlich vor der Wahl, diesen anisotropen Querschnitt in der Herzklappenprothese nachzubilden oder einen rotationssymmetrischen Querschnitt zu verwenden. Die Verwendung eines rotationssymmetrischen Querschnitts für das Grundgerüst einer Mitralklappenprothese hat den Vorteil, dass sich ein derartiges Grundgerüst einfacher auf einen rotationssymmetrischen Katheter aufbringen lässt. Zusätzlich muss die Mitralklappenprothese per Katheter "nur" an die richtige Stelle, der Stelle der natürlichen Mitralklappe, gebracht und expandiert werden. Auf die Orientierung des Katheters und der Mitralklappenprothese bezüglich der Rotationsachse des Katheters bzw. bezüglich des anisotropen Querschnitts der natürlichen Mitralklappe kommt es bei einer derartigen Implantation nicht an. Die Orientierung des Drehwinkels der Rotationsachse oder Hauptachse des Katheters bezüglich einer anisotropen Umgebung wie dem Mitralklappenannulus wird im Folgenden auch als Rotationsorientierung bezeichnet.

Die Implantation einer rotationssymmetrischen Mitralklappenprothese in eine eigentlich anisotrope Umgebung ist jedoch vom funktionellen Standpunkt und dem physiologischen Ergebnis der Prothese nicht optimal.

Die vorliegende Erfindung stellt sich daher die Aufgabe, ein Kathetersystem der eingangs erwähnten Art derart auszugestalten, dass ein anisotropes Implantat in einer anisotropen Umgebung sicher implantiert werden kann. Die Implantation des Implantats soll dabei unter Beachtung der Orientierung des anisotropen Implantats in Bezug auf die Rotationsachse des Kathetersystems und in Bezug auf die anisotropen Gegebenheiten am Implantationsort erfolgen.

Die gestellte Aufgabe wird durch die Merkmalskombination des Anspruches 1 gelöst. Weitere vorteilhafte Ausführungsformen der Erfindung werden in den Unteransprüchen ausgeführt.

Der Grundgedanke der Erfindung basiert auf einer Kombination von Elektrodenkatheter basierten 3-dimensionalen Kartografie- und Navigationssystemen und Kathetersystemen zur Implantation von beispielsweise Herzklappenprothesen. Erfindungswesentlich ist dabei, dass das Kathetersystem zwischen einem Einführzustand und einem Orientierungszustand wechseln kann.

Im Stand der Technik sind Kathetersysteme mit Elektroden bekannt, die zur Diagnose und Ablation von Herzrhythmusstörungen verwendet werden. Derartige Kathetersysteme können mit einem zugehörigen 3-dimensionalen Kartografie- und Navigationssystemen in einem Patienten lokalisiert und navigiert werden. Ein derartiges Ablationskathetersystem offenbart US 6,050,267. Hier wird ein Kathetersystem beschrieben, welches eine Elektrode zur Detektion an der Katheterspitze, eine proximale Ringelektrode und eine Referenzelektrode zur Positionsbestimmung aufweist. Zur 3-dimensionalen Kartografie und Navigation werden dem Patienten noch zusätzlich 3 Elektrodenpaare aufgeklebt. Diese aufgeklebten Elektrodenpaare spannen eine 3-dimensionales Koordinatensystem auf, entlang deren Achsen die Referenzelektrode des Kathetersystems angeregt wird. Über die Wechselwirkung zwischen Anregung und Referenzelektrode des Kathetersystems in Kombination mit einem System zur 3-dimensionalen Bildgebung (z.B. Fluoroskopie) kann der Katheter gesteuert durch den Patienten navigiert und eine Ablation an der gewünschten Stelle durchgeführt werden.

In der US 8,241,274 wird ein ähnliches Kathetersystem mit Sensornavigationsspulen zur präzisen Lokalisation eines Kathetersystems zur Implantation einer Herzklappenprothese in einem 3-dimensionalen Referenzsystem offenbart.

Die bisher im Stand der Technik bekannten Systeme haben jedoch den eingangs beschriebenen Nachteil, dass sie keine Information zur Rotationsorientierung der Rotationsachse des Katheters in Bezug auf das Referenzsystem und damit in Bezug auf den Patienten liefern.

Unter der Rotationsachse des Katheters wird im Rahmen dieser Anmeldung die Hauptachse des Katheters bezeichnet. Diese wird durch die Symmetrieachse der schlauchförmigen Katheterschäfte gebildet. Die Positionsbezeichnungen proximal bzw. distal bezeichnen im Rahmen dieser Anmeldung eine Position nahe dem Behandler bzw. entfernt vom Behandler.

Unter der Rotationsachse oder Hauptachse des Implantats wird die Hauptachse des Grundkörpers verstanden. Dies ist zumeist die Längsachse des Implantats. Bei einer Herzklappenprothese wird im Rahmen dieser Anmeldung beispielsweise die Hauptachse des Grundkörpers der Herzklappenprothese verstanden. Die Hauptachse der Herzklappenprothese stimmt entsprechend bei bestimmungsgemäßer Implantation der Herzklappenprothese mit der Strömungsrichtung des Bluts durch die Herzklappenprothese überein.

Das erfindungsgemäße Kathetersystem kann zwischen einem Einführzustand und einem Orientierungszustand wechseln, wobei dieser Wechsel durch eine Manipulation des Steuergeräts ausgelöst wird und mindestens drei Elektroden des Kathetersystems im Einführzustand einen anderen Raumwinkel aufspannen als im Orientierungszustand.

Wie bereits ausgeführt, soll mit Hilfe des erfindungsgemäßen Kathetersystems ein anisotropes Implantat, insbesondere eine anisotrope Herzklappenprothese, passend in eine anisotrope Implantationsumgebung implantiert werden. Das Kathetersystem selbst ist im Wesentlichen rotationssymmetrisch, lediglich die, auf einem Katheterschaft angeordnete, Herzklappenprothese kann eine Anisotropie aufweisen. Ein Kathetersystem, welches an sich eine gut detektierbare Anisotropie aufweisen würde, wäre schwierig herzustellen und schwer einzuführen.

Unter einem anisotropen Implantat wird im Rahmen der Anmeldung jedes Implantat verstanden, dessen Rotationszustand in Bezug auf die Umgebung am Implantationsort nicht beliebig frei wählbar ist.

Der aufgespannte Raumwinkel wird durch mindestens drei Elektroden bestimmt. Eine Elektrode bildet den Scheitelpunkt des Winkels, die anderen beiden Elektroden bilden Punkte, die auf dem Schenkel des aufgespannten Raumwinkels liegen. Bei Ringelektroden entsprechen die Punkte zur Bestimmung des aufgespannten Raumwinkels dem Mittelpunkt der Ringelektrode. Bei axial orientierten Elektroden können beispielsweise die jeweiligen distalen Endpunkte der Elektroden zur Bestimmung des Raumwinkels verwendet werden.

Der Erfindung liegt der Gedanke zugrunde, ein Kathetersystem mit zwei Zuständen zur Verfügung zu stellen. Das erfindungsgemäße Kathetersystem kann zwischen einem rotationssymmetrischen Einführzustand und einem anisotropen Orientierungszustand wechseln. Die Detektion der Orientierung der Rotationsachse des Implantats, insbesondere der Herzklappenprothese, im 3-dimensionalen Raum wird erfindungsgemäß dadurch gewährleistet, dass sich der von mindestens 3 Elektroden aufgespannte Raumwinkel zwischen Einführzustand und Orientierungszustand ändert. Der Wechsel zwischen Einführzustand und Orientierungszustand und damit die Änderung des von den Elektroden aufgespannten Raumwinkels wird über das Steuergerät ausgelöst.

Das erfindungsgemäße Kathetersystem ermöglicht nicht nur die anatomisch korrekte Positionierung einer anatomisch angepassten, anisotropen Implantats, insbesondere einer Herzklappenprothese, sondern eine exakte Positionierung, die im Zusammenwirken mit einen entsprechend ausgestalteten 3-dimensionalen Kartografie- und Navigationssystem computergestützt oder gänzlich computergesteuert erfolgen kann.

Das erfindungsgemäße System ist insbesondere zur Implantation einer Herzklappenprothese geeignet. Dies gilt insbesondere für eine Herzklappenprothese zum Ersatz der natürlichen Mitralklappe aufweisend einen Grundkörper mit einem D-förmigen Querschnitt wie weiter unten ausgeführt.

Das erfindungsgemäße System ist jedoch ebenfalls vorteilhaft bei Implantaten wie Occluder Devices, Stents für Bifurkationen oder an sich symmetrische Implantate mit einer künstlichen Anisotropie. Ein Implantat mit einer künstlichen Anisotropie könnte beispielsweise auch eine Herzklappenprothese zum Ersatz der natürlichen Aortenklappe mit einem an sich rotationssymmetrischen Grundkörper sein, in den eine Klappenanordnung befestigt ist. Hier kann die Befestigung der Klappenanordnung (beispielsweise über drei über den Umfang verteilte Nähte) für eine Anisotropie sorgen, die an die Implantationsumgebung (den natürlichen Aortenannulus oder den Abgang der Koronar Sinus Arterien) angepasst werden muss. Gleiches gilt, wenn die Rotationsorientierung der Klappenanordnung der Orientierung und natürlichen Lage der Mitral- oder Aortenklappen angepasst werden soll.

Zunächst wird die Herzklappenprothese mit dem Kathetersystem ähnlich dem Stand der Technik bis zum Implantationsort beispielsweise bis an Position der natürlichen Mitralklappe geführt. Das Kathetersystem ist dabei im Einführzustand oder Grundzustand. Wenn die Position der Herzklappenprothese axial zur Katheterachse mit der Position der natürlichen Herzklappe übereinstimmt, wird der Orientierungszustand des Kathetersystems in Bezug auf die anisotrope natürliche Herzklappe des Patienten festgestellt. Das Kathetersystem wechselt vom Einführzustand in den Orientierungszustand, der von mindestens 3 Elektroden aufgespannte Raumwinkel ändert sich und die Orientierung der anisotropen Herzklappenprothese in Bezug auf die anisotrope natürliche Klappe kann detektiert werden. Der Wechsel vom Einführzustand auf den Orientierungszustand kann dabei bestimmt werden, wenn sich die Herzklappenprothese am Implantationsort oder proximal oder distal davon befindet.

Die Detektion der Änderung des Raumwinkels kann über 3-dimensionale Kartografiesysteme erfolgen, wie sie im Stand der Technik, beispielsweise in US 6,050,267, bekannt sind. Da die Positionierung der anisotropen Herzklappenprothese und die Änderung des Raumwinkels fest und bekannt sind, kann über die Detektion der Raumwinkeländerung direkt auf die Orientierung der anisotropen Herzklappenprothese in Bezug auf die anisotrope Implantationsumgebung geschlossen werden. Die im erfindungsgemäßen Kathetersystem vorhanden Elektroden ermöglichen im Zusammenspiel mit dem 3-dimensionalen Kartografiesystem (beispielsweise mittels 3 Paar verschiedener Referenzelektroden, die auf dem Patienten angebracht sind) die Detektion der Änderung des elektrischen Dipols im Referenzsystem. Durch den vorgegebenen Zusammenhang zwischen Einführzustand und Orientierungszustand kann aus der Änderung der räumlichen Orientierung des elektrischen Dipols direkt auf die Orientierung des Kathetersystems bzw. die Orientierung der Rotationsachse des Kathetersystems geschlossen werden. Die asymmetrische Bauform und Anordnung der mindestens drei Elektroden ermöglicht es erst, eine räumliche Anordnung derselben zu messtechnisch detektieren. Ähnlich funktioniert das System bei der Verwendung eines Magnetfeldes anstatt der aufgeklebten Elektrodenpaare als Referenzsystem.

Der Wechsel zwischen Einführzustand und Orientierungszustand ist bevorzugt reversibel und beliebig oft wiederholbar.

Bevorzugt ist im Orientierungszustand mindestens eine Elektrode nicht mehr auf der Hauptachse des Kathetersystems im Einführzustand angeordnet. In dieser bevorzugten Ausgestaltung weist das Kathetersystems drei Elektroden, von proximal nach distal angeordnet und voneinander beabstandet, auf. Im Einführzustand befinden sich alle 3 Elektroden auf der Rotations- und Hauptachse des Kathetersystems. Im Orientierungszustand wird eine dieser Elektroden von der Hauptachse des Kathetersystems weg bewegt. Durch den bekannten Zusammenhang zwischen Bewegungsrichtung der Elektrode vom Einführzustand in den Orientierungszustand und der Orientierung des anisotropen Implantats, insbesondere der Herzklappenprothese, bezüglich der Hauptachse des Katheters und der Bewegungsrichtung kann so eindeutig auf die Orientierung der Hauptachse des Kathetersystems bzw. der Hauptachse des Implantats, insbesondere der Herzklappenprothese, im Referenzsystem und damit im Patienten geschlossen werden. In einfachsten Fall wird beispielsweise ein Teil des Katheterschafts mit mindestens einer Elektrode gegen den restlichen Katheterschaft mit den restlichen Elektroden verknickt, um vom Einführzustand in den Orientierungszustand zu wechseln. Diese Verknickung erfolgt mit definiertem Winkel und, in Bezug auf das anisotrope Implantat, insbesondere die anisotrope Herzklappenprothese, in definierter Richtung. Entsprechend ändert sich der von mindestens drei Elektroden aufgespannte Raumwinkel in definierter Weise.

In einer vorteilhaften Ausgestaltung der Erfindung sind die Elektroden als Ringelektroden ausgeführt und die Elektrodenleitungen in einem Katheterschaft eingebettet sind. Ebenso zweckmäßig wären aber auch einzelne Lumen für die Elektrodenleitungen. Unter einer Ringelektrode wird im Rahmen der Erfindung ein elektrischer Leiter verstanden, der kreisförmig um die Hauptachse des Kathetersystems, bevorzugt auf einem Katheterschaft, angeordnet ist. Eine Ringelektrode ist daher vereinfacht ausgedrückt, ein Ring aus elektrisch leitenden Material um einen Katheterschaft. Unter einer Elektrodenleitung wird die entsprechend elektrische Kontaktierung der Elektrode mit einer möglichen Spannungsquelle oder Analyse-/Steuereinheit proximal und außerhalb des Patienten bei bestimmungsgemäßen Gebrauch des Kathetersystems verstanden. Derartige Elektrodenanordnungen lassen sich besonders einfach von einem Einführzustand in einen Orientierungszustand überführen. Vorteilhafterweise kann jedoch auch eine der Elektroden als distale Elektrode am distalen Ende des Kathetersystems (so genannte Tip-Elektrode) ausgeführt werden.

In einer Ausgestaltung sind mindestens zwei Elektroden auf zwei verschiedenen Katheterschäften angeordnet sind, deren Hauptachsen im Einführzustand parallel verlaufen und die bevorzugt zumindest teilweise miteinander fest verbunden sind. In dieser Ausgestaltung der Erfindung weist das Kathetersystem mindestens zwei Katheterschäfte auf, die zumindest teilweise miteinander verbunden sind und deren Hauptachsen sowohl zueinander parallel sind als auch parallel zur Hauptachse des Kathetersystems in seinem Einführzustand sind. In dieser Ausgestaltung der Erfindung erfolgt der Wechsel zwischen Einführzustand und Orientierungszustand des Kathetersystems dadurch, dass die beiden Katheterschäfte mit jeweils mindestens einer Elektrode gegeneinander auf definierte Art und Weise bewegt werden. Auch hier kann wieder in der einfachsten Ausgestaltung ein Katheterschaft gegen den anderen Katheterschaft abgeknickt werden, wodurch sich der von den Elektroden insgesamt umfasste Raumwinkel ändert.

Bei einem derartigen System mit parallelen Katheterschäften können diese selbst zusätzlich auch noch mehrere ineinander angeordnete Katheterschäfte aufweisen, die gegeneinander beweglich sind.

Zweckmäßigerweise weist die Herzklappenprothese ein Grundgerüst und eine Klappenanordnung auf, wobei das Grundgerüst aus einem selbstexpandierenden oder ballonexpandierbaren Material besteht und das Grundgerüst bevorzugt einen anisotropen, besonders bevorzugt einen D-förmigen, Querschnitt aufweist. Das Grundgerüst wird in dieser Ausgestaltung der Erfindung von seiner komprimierten Form im Einführzustand am Implantationsort expandiert und so an Stelle der natürlichen Klappe verankert. Die natürlichen Herzklappen werden dabei zumeist vom Grundgerüst verdrängt. Denkbar ist jedoch auch eine minimal invasive Entfernung der natürlichen Klappen. Die Klappenanordnung ist im Grundgerüst fixiert und über dieses an der Stelle der natürlichen Klappe verankert. Die Klappenanordnung übernimmt die Klappenfunktion der natürlichen Klappe und kann entsprechend zwischen einem offenen und einem geschlossenen Zustand in Bezug auf die natürliche Richtung des Blutstroms wechseln.

Der Wechsel zwischen komprimierten Zustand und expandierten, implantierten Zustand der Herzklappenprothese kann dabei entweder über eine Ballondilatation erfolgen oder das Grundgerüst wird aus selbstexpandierendem Material ausgeführt. In diesem Fall wird das Grundgerüst mittels einer mechanischen Kraft (z.B. eine die Herzklappenprothese überdeckende Kapsel) in seinem komprimierten Zustand gehalten. Nach Wegfall der Haltekraft (z.B. durch proximales Zurückziehen der die Herzklappenprothese überdeckenden Kapsel) expandiert das Grundgerüst und damit die Herzklappenprothese automatisch und verankert sich an der Stelle der natürlichen Klappe.

In einer weiteren bevorzugten Ausgestaltung weist ein erster Katheterschaft ein Lumen für einen Führungsdraht auf und trägt das Implantat, insbesondere die Herzklappenprothese, während ein zweiter Katheterschaft den ersten Katheterschaft umgibt und mit seinem distalen Bereich das Implantat, insbesondere die Herzklappenprothese, überdeckt. Diese Ausgestaltung kommt vor allem für selbstexpandierende Implantate, insbesondere eine Herzklappenprothese, mit einem selbstexpandierenden Grundgerüst in Frage. Der distale Teil des zweiten Katheterschafts (häufig im Stand der Technik als Kapsel bezeichnet) umgibt die Herzklappenprothese und hält diese während des Einführzustand im komprimierten Zustand. Im Einführzustand wird das Kathetersystem im Patienten soweit vorgeschoben, bis sich die Herzklappenprothese an der Stelle der natürlichen Herzklappe befindet. Das Kathetersystem wechselt nun von seinem Einführzustand auf seinen Orientierungszustand und die Rotationsorientierung der Hauptachse des Kathetersystems und damit die Rotationsorientierung der Hauptachse der Herzklappenprothese bestimmt wird. Stimmt die Rotationsorientierung der anisotropen Herzklappenprothese mit der anisotropen Implantationsumgebung überein, wird der zweite Katheterschaft nach proximal zurückgezogen und die Herzklappenprothese frei gegeben. Das Grundgerüst der Herzklappenprothese expandiert und verankert sich dadurch am Implantationsort.

In einer anderen Ausgestaltung weist ein erster Katheterschaft ein Lumen für einen Führungsdraht auf und ein zweiter Katheterschaft umgibt den ersten Katheterschaft, wobei der zweite Katheterschaft ein Lumen für ein Fluid und einen dilatierbaren Ballon an seinem distalen Ende aufweist, wobei das Implantat, insbesondere die Herzklappenprothese, über dem Ballon angeordnet und mittels Ballondilatation expandierbar ist. Diese Ausgestaltung funktioniert in Bezug auf die Feststellung der Rotationsorientierung genau wie die vorangehend beschriebene Ausgestaltung. In dieser Ausgestaltung wird jedoch das Grundgerüst des Implantats bzw. der Herzklappenprothese mittels Dilatation eines darunter befindlichen Ballons expandiert und am Implantationsort verankert. Die Fluidbeaufschlagung erfolgt über das dafür vorgesehene Lumen des zweiten Katheterschafts.

Besonders bevorzugt ist die Herzklappenprothese zur Implantation an Stelle der Mitralklappe geeignet. Die Vorteile der Erfindung kommen besonders bei einer Herzklappenprothese mit einem anisotropen Grundgerüst, insbesondere einem Grundgerüst mit einem D-förmigen Querschnitt, zum Ersatz der natürlichen Mitralklappe zum Tragen. Besonders bevorzugt ist die Herzklappenprothese daher als Mitralklappenprothese mit einem Grundgerüst ausgestaltet, wobei das Grundgerüst einen D-förmigen Querschnitt aufweist. Unter einem D-förmigen Querschnitt wird im Rahmen dieser Anmeldung ein Querschnitt verstanden, der einen annähernd geraden Abschnitt und einen gebogenen (bevorzugt kreisförmig oder elliptischen) Abschnitt aufweist.

Bevorzugt ist das Steuergerät zur automatischen Steuerung des Kathetersystems in Zusammenwirkung mit einem 3-D Kartografiesystem eines Patienten ausgebildet ist. In dieser Ausgestaltung der Erfindung erfolgt die Implantation der Herzklappenprothese automatisch mittels Computersteuerung. Hier wird nicht nur computergestützt das Kathetersystem und die darauf angeordnete Herzklappenprothese bis an den Implantationsort geführt sondern auch automatisch zwischen Einführzustand und Orientierungszustand gewechselt. Je nach bestimmter Rotationsorientierung des Kathetersystems / der Herzklappenprothese wird die Rotationsorientierung korrigiert und die Herzklappenprothese am Implantationsort expandiert und implantiert.

Mit der vorliegenden Erfindung gelingt es insbesondere eine (anisotrope) Herzklappenprothese rotationsorientierungsgenau am Zielort zu implantieren. Das Kathetersystem hat dabei alle Voraussetzungen vor eine computergestützte, automatische Implantation.

Im Folgenden soll die Erfindung anhand der in den Figuren dargestellten Ausführungsbeispiele näher erläutert werden.

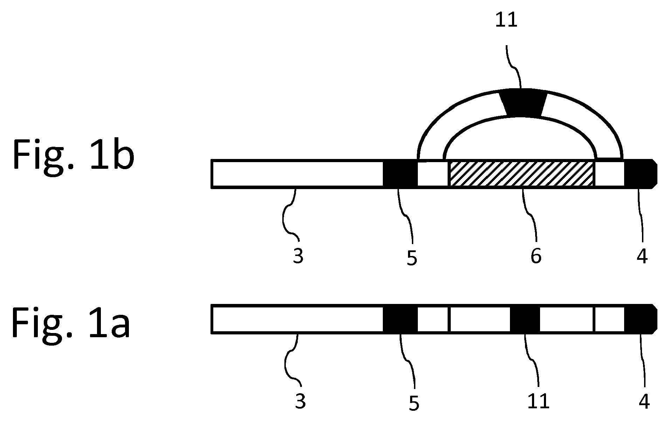

In den Figuren 1 bis 6 werden sechs Ausführungsbeispiele eines Kathetersystems im Sinne der vorliegenden Erfindung schematisch dargestellt. Dabei zeigen die Figuren 1b, 2b, 3b, 4b, 5b und 6b jeweils den Orientierungszustand des Kathetersystems, während die Figuren 1a, 2a, 3a, 4a, 5a und 6a den Einführzustand des Kathetersystems beschreiben. Gleiche Bauteile weisen in allen Figuren die gleichen Bezugszeichen auf.

Figur 1 zeigt schematisch ein erstes Ausführungsbeispiel eines erfindungsgemäßen Kathetersystems in seinem Einführzustand (Fig. 1a) und seinem Orientierungszustand (Fig. 1b). Das Kathetersystem besteht aus zwei verschiedenen Katheterschäften, deren Hauptachsen im Wesentlichen parallel verlaufen und die zumindest teilweise miteinander verbunden sind. Im Einführzustand (Fig. 1a) ist im Wesentlichen nur ein Katheterschaft 3 dargestellt, welcher eine proximale Ringelektrode 5 und eine distal davon angeordnete Orientierungselektrode 11 aufweist, welche ebenfalls als Ringelektrode ausgeführt ist. Der zweite, parallel angeordnete und mit dem ersten Schaft verbundene Katheterschaft trägt die Herzklappenprothese, beispielsweise eine Mitralklappenprothese, 6 und eine distale Ringelektrode 4.

Im Einführzustand sind die Hauptachsen der beiden Katheterschäfte parallel zur Hauptachse des Kathetersystems. Im Orientierungszustand (Fig. 1b) wird die Orientierungselektrode 11 durch eine bogenförmige Verkrümmung des Katheterschafts ausgelenkt. Entsprechend ändert sich der Raumwinkel der von der proximalen Ringelektrode 5, der Orientierungselektrode 11 und der distalen Ringelektrode 4 aufgespannt wird. Aus dieser Änderung des aufgespannten Raumwinkels kann über ein entsprechendes Referenzsystem (nicht dargestellt) die Rotationsorientierung der Herzklappenprothese 6 bestimmt werden. Nach entsprechender Anpassung der Rotationsorientierung kann die Herzklappenprothese 6 beispielsweise über eine Ballondilatation expandiert und implantiert werden.

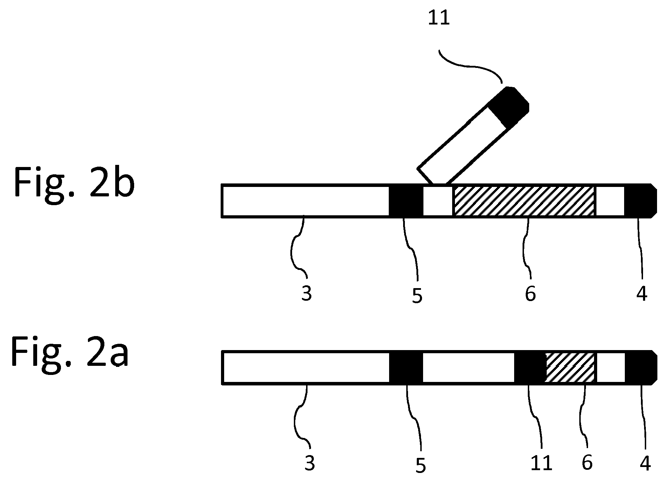

Figur 2 zeigt ebenfalls schematisch ein Ausführungsbeispiel der Erfindung, wobei hier der Katheterschaft 3 mit der Orientierungselektrode 11 kürzer ist als der Katheterschaft, der die Herzklappenprothese 6 und die distale Ringelektrode 4 trägt. Im Orientierungszustand (Fig. 1b) wird das distale Ende des Katheterschafts 3 mit der Orientierungselektrode 11 abgeknickt.

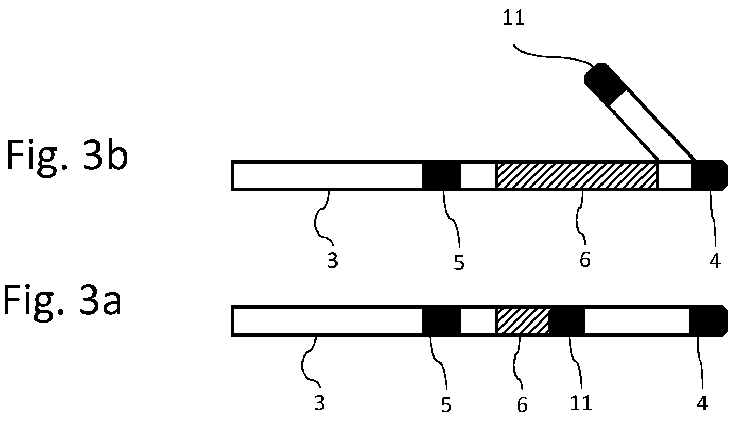

Das Ausführungsbeispiel in Figur 3 unterscheidet sich vom Ausführungsbeispiel nach Figur 2 dadurch, dass das Abknicken der Orientierungselektrode 11 vom distalen Ende des Katheterschafts her erfolgt.

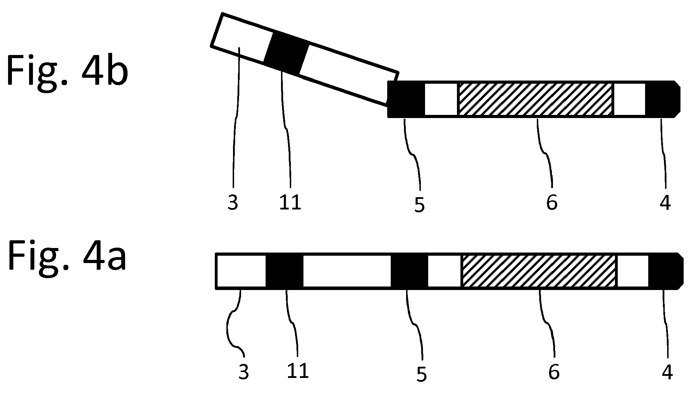

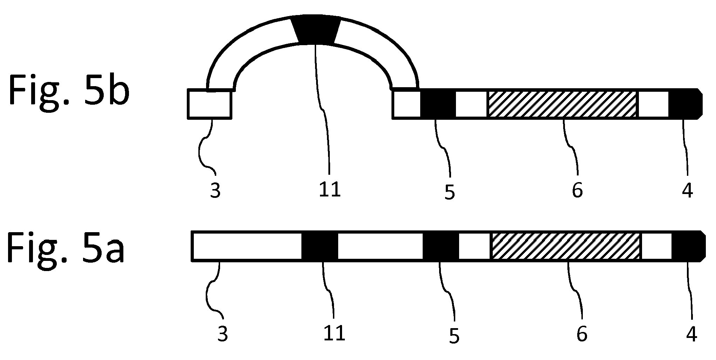

Die Ausführungsbeispiele der Figuren 4 bis 6 sind Ausführungsbeispiele ohne parallel angeordnete Katheterschäfte. Im Ausführungsbeispiel nach Figur 4 und 5 befindet sich die Orientierungselektrode 11 proximal der Ringelektroden 5 und 4. Hier wird eine Änderung des Raumwinkels aufgespannt durch die Orientierungselektrode 11, die proximale Ringelektrode 5 und die distale Ringelektrode 4 durch ein Abknicken (Fig. 4b) oder ein Aufspannen (Figur 5b) des proximalen Ende des Katheterschafts 3 erreicht.

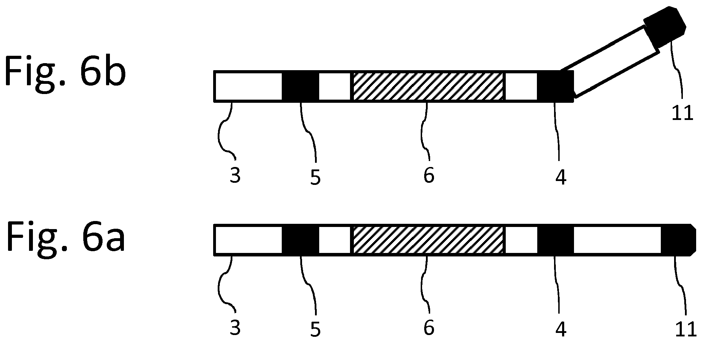

Figur 6 zeigt ein Ausführungsbeispiel eines Kathetersystems mit einer selbstexpandierenden Herzklappenprothese. Die Herzklappenprothese 6 ist von einem zweiten Katheterschaft 3 umgeben und wird von diesem im Einführzustand (Fig. 6a) im komprimierten Zustand gehalten. Zwei Ringelektroden sind proximal (Ringelektrode 5) und distal (Ringelektrode 4) der Herzklappenprothese angeordnet. Beim Wechsel vom Einführzustand (Fig. 6a) in den Orientierungszustand (Fig. 6b) knickt entweder das ganze Kathetersystem mit Katheterschaft 3 und darin angeordneten Innenschaft (erster Katheterschaft, der die Herzklappenprothese trägt) oder nur der Innenschaft distal der Herzklappenprothese und der distalen Ringelektrode 4 ab. Wenn nur der Innenschaft abknickt, ist der Außenschaft zweckmäßigerweise kürzer ausgeführt als der Innenschaft. Durch Abknicken des distalen Endes mit der Orientierungselektrode 11 hat sich der aufgespannte Raumwinkel von Ringelektrode 5, Ringelektrode 4 und Orientierungselektrode geändert. Nach Einstellung der Rotationsorientierung der Herzklappenprothese 6 im Orientierungszustand wird dieser verlassen und die Herzklappenprothese 6 durch Rückzug des Außenschafts 3 freigesetzt und implantiert.

## Patentansprüche

1. Kathetersystem umfassend mindestens einen Katheterschaft, ein anisotropes Implantat (6), ein Steuergerät zur Steuerung und Manipulation des Katheters sowie mindestens drei Elektroden (4, 5, 11) im distalen Bereich des Katheters, die über jeweils eine Elektrodenleitung leitend mit einer Analyseeinheit verbunden sind, **dadurch gekennzeichnet, dass** das Kathetersystem zwischen einem Einführzustand und einem Orientierungszustand wechseln kann, dieser Wechsel durch eine Manipulation des Steuergeräts ausgelöst wird und mindestens drei Elektroden (4, 5, 11) des Kathetersystems im Einführzustand einen anderen Raumwinkel aufspannen als im Orientierungszustand.

2. Kathetersystem nach Anspruch 1, **dadurch gekennzeichnet, dass** das anisotrope Implantat als Herzklappenprothese (6) ausgebildet ist.

3. Kathetersystem nach Anspruch 1 oder 2, **dadurch gekennzeichnet, dass** im Orientierungszustand mindestens eine Elektrode (11) nicht mehr auf der Hauptachse des Kathetersystems im Einführzustand angeordnet ist.

4. Kathetersystem nach einem der Ansprüche 1 bis 3, **dadurch gekennzeichnet, dass** die Elektroden (4, 5, 11) als Ringelektroden ausgeführt und die Elektrodenleitungen in einem Katheterschaft eingebettet sind.

5. Kathetersystem nach einem der Ansprüche 1 bis 4, **dadurch gekennzeichnet, dass** mindestens zwei Elektroden auf zwei verschiedenen Katheterschäften angeordnet sind, deren Hauptachsen im Einführzustand parallel verlaufen und die bevorzugt zumindest teilweise miteinander fest verbunden sind.

6. Kathetersystem nach einem der Ansprüche 2 bis 5, **dadurch gekennzeichnet, dass** die Herzklappenprothese (6) ein Grundgerüst und eine Klappenanordnung aufweist, wobei das Grundgerüst aus einem selbstexpandierenden oder ballonexpandierbaren Material besteht und das Grundgerüst bevorzugt einen anisotropen, besonders bevorzugt einen D-förmigen, Querschnitt aufweist.

7. Kathetersystem nach einem der Ansprüche 2 bis 6, **dadurch gekennzeichnet, dass** ein erster Katheterschaft ein Lumen für einen Führungsdraht aufweist und die Herzklappenprothese (6) trägt und ein zweiter Katheterschaft den ersten Katheterschaft umgibt und mit seinem distalen Bereich die Herzklappenprothese überdeckt.

8. Kathetersystem nach einem der Ansprüche 2 bis 6, **dadurch gekennzeichnet, dass** ein erster Katheterschaft ein Lumen für einen Führungsdraht aufweist und ein zweiter Katheterschaft den ersten Katheterschaft umgibt, wobei der zweite Katheterschaft ein Lumen für ein Fluid und einen dilatierbaren Ballon an seinem distalen Ende in Kommunikation mit dem Fluid aufweist, wobei die Herzklappenprothese (6) über dem Ballon angeordnet und mittels Ballondilatation expandierbar ist.

9. Kathetersystem nach einem der Ansprüche 2 bis 8, **dadurch gekennzeichnet, dass** die Herzklappenprothese (6) zur Implantation an Stelle der Mitralklappe geeignet ist.

10. Kathetersystem nach einem der vorangehenden Ansprüche, **dadurch gekennzeichnet, dass** das Steuergerät zur automatischen Steuerung des Kathetersystems in Zusammenwirkung mit einem 3-D Kartografiesystem eines Patienten ausgebildet ist.
